Europäisches Patentamt

European Patent Office

Office européen des brevets

(11) Veröffentlichungsnummer : **0 121 760**
**B1**

# (12) EUROPÄISCHE PATENTSCHRIFT

(45) Veröffentlichungstag der Patentschrift :
28.05.86

(21) Anmeldenummer : **84102367.4**

(22) Anmeldetag : **05.03.84**

(51) Int. Cl.⁴ : **C 07 D307/20**

(54) Verfahren zur Herstellung von 3-Hydroxytetrahydrofuran.

(30) Priorität : **12.03.83 DE 3308931**

(43) Veröffentlichungstag der Anmeldung :
**17.10.84 Patentblatt 84/42**

(45) Bekanntmachung des Hinweises auf die Patenterteilung : **28.05.86 Patentblatt 86/22**

(84) Benannte Vertragsstaaten :
**BE CH DE FR GB IT LI**

(56) Entgegenhaltungen :
EP-B- 0 001 291
DE-A- 2 649 900
DE-C- 841 592
ORGANIC SYNTHESIS, Collective, Vol. 4, New York 1963, H. WYNBERG et al. "3 Hydroxytetrahydrofuran", Seiten 534,535
JUSTUS LIEBIGS ANNALEN DER CHEMIE, Band 596, 1955, Weinheim, REPPE UND MITARBEITER "Äthinylierung V", Seiten 108-113

(73) Patentinhaber : **BASF Aktiengesellschaft**
**Carl-Bosch-Strasse 38**
**D-6700 Ludwigshafen (DE)**

(72) Erfinder : **Mueller, Herbert, Dr.**
**Carostrasse 53**
**D-6710 Frankenthal (DE)**
Erfinder : **Voges, Dieter, Dr.**
**Richard-Wagner-Strasse 28**
**D-6800 Mannheim 1 (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Diese Erfindung betrifft ein Verfahren zur Herstellung von 3-Hydroxytetrahydrofuran durch Dehydratisierung von 1,2,4-Butantriol.

3-Hydroxytetrahydrofuran ist ein wertvolles Zwischenprodukt für Wirkstoffsynthesen. Es wird bekanntlich aus 1,2,4-Butantriol durch Wasserabspaltung mit sauren Katalysatoren hergestellt. Da es sich beim 1,2,4-Butandiol um ein Folgeprodukt des Buten-2-diols-1,4 handelt, das ebenfalls sehr wertvoll ist, ist es für eine wirtschaftliche Herstellung von 3-Hydroxytetrahydrofuran dringend geboten, die Dehydratisierung mit möglichst hoher Ausbeute durchzuführen.

Nach den Angaben der Literatur werden bei der Dehydratisierung von 1,2,4-Butantriol Ausbeuten von 80 bis 95 Mol.% erhalten (s. Organis Syntheses, New York, 1983 Collect. Vol. 4, Seiten 534 bis 535 ; Annalen der Chemie, Band 596 (1955), Seite 112 und DE-PS-841 592). Die bei der technischen Durchführung dieser Verfahren erzielbaren Ausbeuten gehen jedoch über 90 Mol.% nicht hinaus.

Ziel der Erfindung war es, ein Verfahren zu finden, das es ermöglicht, bei der Herstellung von 3-Hydroxytetrahydrofuran durch Dehydratisierung von 1,2,4-Butantriol Ausbeuten von mehr als 95 Mol.% zu erzielen.

Diese Aufgabe wurde durch das erfindungsgemäße Verfahren gelöst. Nach dem neuen Verfahren stellt man 3-Hydroxytetrahydrofuran durch Dehydratisierung von 1,2,4-Butantriol in flüssiger Phase an einem Katalysator bei höherer Temperatur so her, daß man einen Katalysator aus der Gruppe der Bleicherden verwendet, und die Dehydratisierung in Gegenwart einer mindestens 3-fachen molaren Menge an Wasser, bezogen auf das 1,2,4-Butantriol, vornimmt.

Man führt die Dehydratisierung des 1,2,4-Butantriol in flüssiger Phase mit dem suspendierten oder fest angeordneten Katalysator bei höheren Temperaturen durch. Die Reaktionsgeschwindigkeit hängt ab von der gewählten Reaktionstemperatur und der Katalysatormenge. Bei 165 °C z. B. gelingt es je Gewichtsteil Katalysator bis zu 5 Teile 3-Hydroxytetrahydrofuran je Stunde aus 1,2,4-Butantriol herzustellen. Man führt die Dehydratisierung zweckmäßig zwischen 150 und 200 °C, vorzugsweise zwischen 150 und 180 °C durch. Im allgemeinen arbeitet man bei gewöhnlichem, d. h. atmosphärischem oder geringfügig höherem oder niedrigerem Druck. Für die Erreichung des hohen Ausbeuteizieles ist es nicht erforderlich, den geringen Druck von 30 Torr und weniger, wie er für das in der DE-PS-841 592 bekannte Verfahren gefordert wird, einzuhalten. Auch durch diese Vereinfachung hebt sich das erfindungsgemäße Verfahren vorteilhaft vom Stande der Technik ab.

Nach dem erfindungsgemäßen Verfahren verwendet man Katalysatoren aus der Gruppe der Bleicherden. Bleicherden, auch Fullererden genannt, sind kolloidale, wasserhaltige Aluminiumhydrosilikate aus der Montmorillonit-Gruppe, bei denen die Aluminiumionen teilweise durch Eisen- oder Magnesiumionen ersetzt sein können. Das Verhältnis von Kieselsäure zu Tonerde in diesen Mineralien beträgt etwa 4 : 1. Es sind handelsübliche Produkte, die durch Säurebehandlung aktiviert sind und in großen Umfange zur Raffination von Speiseölen und Fetten sowie Mineralölen verwendet werden.

Die Menge an Bleicherde, bezogen auf das Butan-1,2,4-diol, beträgt z. B. 0,1 bis 30, vorzugsweise 5 bis 20 Gew.%.

Beim Einsatz der genannten Katalysatoren kann ein Zusatz von Carbonaten der Alkali- oder Erdalkalimetalle vorteilhaft sein. Die Menge an Alkali- oder Erdalkalimetallcarbonat bzw. an den entsprechenden Bicarbonaten, die man zusetzt, beträgt 0,1 bis 1, vorteilhaft 0,3 bis 0,9 Gew.%, bezogen auf das Gewicht des Katalysators. Je nach Provenienz des Katalysators kann unter Umständen auf die Dotierung mit den genannten Alkali- oder Erdalkaliverbindungen verzichtet werden. Ob sich der Zusatz empfiehlt oder nicht, wird am besten durch ein Experiment im Laboratorium bestimmt.

Um das Ziel der Erfindung zu erreichen, ist es unerläßlich, die Dehydratisierung in Gegenwart einer mindestens 3-fachen molaren Menge, bezogen auf das 1,2,4-Butantriol an Wasser durchzuführen. Zweckmäßigerweise gibt man eine 5 bis 20-fache molare Menge an Wasser zu. Das Ziel wird nicht erreicht, wenn außer dem Wasser, das durch die Dehydratisierung entsteht, kein Zusatz an Wasser erfolgt. Da die Reaktionstemperatur gewöhnlich über der Siedetemperatur des Wassers liegt, kann die notwendige Wassermenge in Form von Wasserdampf zugeführt werden. Als besonders vorteilhaft hat es sich aber erwiesen — und dieser Umstand ist nicht ohne weiteres zu verstehen — wenn man das Wasser in Form einer Lösung des Butantriols dem Reaktionsgemisch zuführt. Da man Ausbeuten von über 90 Mol.% nur beim Einsatz von solchen wäßrigen Butantriol-Lösungen erhält, die mindestens 60 Mol.% Wasser enthalten, wird man im allgemeinen Lösungen einsetzen, die aus 40 und weniger Mol.% Butantriol und 60 und mehr Mol.% Wasser bestehen. Im allgemeinen wird man aus Gründen der Wirtschaftlichkeit keine wäßrigen Lösungen verwenden, die weniger als 5 Mol.% Butantriol enthalten.

Das Verfahren kann zwar absatzweise betrieben werden, jedoch ist die fortlaufende Betriebsweise von Vorteil. Besonders zweckmäßig ist es, wenn man das umzusetzende Butantriol als wäßrige Lösung unter Rühren in dem Maße zugibt, in dem das Reaktionsprodukt und das Reaktionswasser sowie das zusätzlich verwendete Wasser als Reaktionshilfe aus dem Reaktionsgefäß, z. B. über eine entsprechende Fraktionierkolonne abdestillieren. In diesem Falle erhält man am Kolonnenkopf in der Regel das reine Produkt zusammen mit dem gebildeten und dem mit dem Ausgangsprodukt zugeführten Wasser.

Nach dem neuen Verfahren erhält man 3-Hydroxytetrahydrofuran in praktisch quantitativer Ausbeute. Das Verfahren zeichnet sich auch durch eine beliebig hohe Katalysatorproduktivität aus und ist besonders umweltfreundlich. Da das Reaktionsgemisch keine korrosiven Substanzen enthält, lassen sich Apparate aus billigen Werkstoffen, wie aus gewöhnlichem Apparatestahl verwenden. Auf die Anwendung verbleiter Behälter, die bei der üblichen Durchführung der Reaktion mit Schwefelsäure oder auch Sulfonsäuren nötig und technisch gebräuchlich sind, kann verzichtet werden. Dieses vorteilhafte Verfahrensergebnis war nicht zu erwarten. Arbeitet man nämlich entsprechend den Angaben in der EP-PS-1 291, in der ein Verfahren zur Herstellung von Tetrahydrofuran durch Dehydratisierung von Diolen an Bleicherden beschrieben wird, so erhält man 3-Hydroxytetrahydrofuran aus 1,2,4-Butantriol in Ausbeuten von nur etwa 60 Mol.%.

Da sich bei der Cyclisierung zum Hydroxytetrahydrofuran ein Molekül Wasser bildet, war anzunehmen, daß die Dehydratisierung durch eine schnelle Entfernung des Reaktionswassers gefördert wird. Überraschenderweise stellte sich jedoch heraus, daß die Synthese sowohl in der Selektivität als auch der Geschwindigkeit durch die Anwesenheit zusätzlicher Wassermengen gefördert wird. So erzielt man z. B. mit einem Überschuß an Wasser im Zulaufprodukt zum Dehydratisierungsreaktor, der 90 Mol.% und mehr beträgt, Ausbeuten an Hydroxytetrahydrofuran die zwischen 96 und 100 Mol.% liegen. Im Vergleich zur Dehydratisierung in Abwesenheit bzw. in Anwesenheit von geringen Wassermengen wird nach der erfindungsgemäßen Arbeitsweise eine Vervielfältigung der Umsetzungsgeschwindigkeit erreicht.

Die in dem folgenden Beispiel genannten Teile sind Gew.-Teile, sie verhalten sich zu Volumina wie kg zu Liter.

Beispiel

In eine Destillationsblase, die mit Rührwerk und einem Fraktionieraufsatz (5 theoretische Böden) versehen ist, gibt man 40 Teile 1,2,4-Butantriol, 10 Teile Bleicherde, die im Handel unter der Bezeichnung (R)Tonsil, Optimum FF (Hersteller: Süd-Chemie, München) erhältlich ist und 0,01 Teile Natriumbicarbonat. Man gibt dann noch 50 Teile Wasser hinzu (das ist die 7,4-fache molare Menge, bezogen auf das Butantriol) und erhitzt das Gemisch auf 165 °C. Bereits bei 150 °C setzt lebhafte Reaktion ein. Bei 165 °C werden stündlich 20 Teile Butantriol zu 3-Hydroxytetrahydrofuran umgesetzt. Diese erscheinen zusammen mit dem Wasser am Kopf der Fraktionierkolonne bei einer Siedetemperatur von ca. 130 °C und werden als wäßrige Lösung dem Reaktionssystem entnommen. Im Maße der Umsetzungsgeschwindigkeit wird in der Blase das verbrauchte Butantriol und verdampfte Wasser in Form einer 40 %igen wäßrigen Butantriol-Lösung durch neuen Zulauf ergänzt. Nachdem 2 000 Teile Butantriol umgesetzt sind, beträgt die Reaktionsgeschwindigkeit immer noch 30 % der ursprünglich festgestellten Reaktionsgeschwindigkeit. Die Reaktion wird nun unterbrochen bzw. zu Ende geführt, indem im Verlaufe von 5 Stunden 100 Teile Wasser in die Blase der Destillationseinrichtung bei 170 °C zugegeben werden. In der Destillationsblase bleiben dann neben der eingesetzten Bleicherde ca. 0,5 bis 1 Gew.% Höhersieder, bezogen auf das zugeführte Butantriol in der Destillationsblase zurück. Durch fraktionierte Destillation im Vakuum mit Hilfe einer Fraktionierkolonne (10 theoretische Böden) läßt sich das 3-Hydroxytetrahydrofuran mit einer Ausbeute von über 98 Mol.%, bezogen auf das ursprünglich eingesetzte Triol, mit einer Reinheit (GC) von über 99,8 Fl-% gewinnen.

**Patentansprüche**

1. Verfahren zur Herstellung von 3-Hydroxytetrahydrofuran durch Dehydratisierung von 1,2,4-Butantriol in flüssiger Phase an einem Katalysator bei höherer Temperatur, dadurch gekennzeichnet, daß man einen Katalysator aus der Gruppe der Bleicherden verwendet, und die Dehydratisierung in Gegenwart einer mindestens 3-fachen molaren Menge an Wasser, bezogen auf das 1,2,4-Butantriol, vornimmt.

2. Verfahren nach Anspruch 1, dadurch gekennzeichnet, daß man zum Ausgangsgemisch 0,05 bis 1 Gew.%, bezogen auf die Bleicherde, eines Carbonates der Alkali- oder Erdalkalimetalle gibt.

3. Verfahren nach Anspruch 1, dadurch gekennzeichnet, daß man die Dehydratisierung bei Temperaturen von 150 bis 200 °C unter Abdestillieren eines Gemisches aus 3-Hydroxytetrahydrofuran und Wasser und gleichzeitigem Zuführen einer wäßrigen Lösung von 1,2,4-Butantriol, die mindestens 60 % Wasser enthält, durchführt.

**Claims**

1. A process for the preparation of 3-hydroxytetrahydrofuran by dehydration of butane-1,2,4-triol in the liquid phase over a catalyst at elevated temperature, wherein a catalyst selected from the group of the bleaching earths is used, and the dehydration is carried out in the presence of not less than 3 moles of water per mole of butane-1,2,4-triol.

2. A process as claimed in claim 1, wherein from 0.05 to 1 % by weight, based on the bleaching earth, of an alkali metal carbonate or alkaline earth metal carbonate is added to the starting mixture.

3. A process as claimed in claim 1, wherein the dehydration is carried out at from 150 to 200 °C, a mixture of 3-hydroxytetrahydrofuran and water being distilled off, and an aqueous solution of butane-1,2,4-triol, containing not less than 60 % of water, being introduced at the same time.

**Revendications**

1. Procédé de préparation de l'hydroxy-3 tétrahydrofuranne par déshydratation du butane-triol-1,2,4 en phase liquide, à température accrue, en présence d'un catalyseur, caractérisé en ce que le catalyseur est choisi dans le groupe des terres décolorantes et la déshydratation est réalisée en présence d'une proportion d'eau au moins trois fois molaire par rapport au butane-triol-1,2,4.

2. Procédé suivant la revendication 1, caractérisé en ce que l'on incorpore au mélange de départ entre 0,05 et 1 % du poids de la terre décolorante d'un carbonate d'un métal alcalin ou alcalino-terreux.

3. Procédé suivant la revendication 1, caractérisé en ce que la déshydratation est effectuée entre 150 et 200 °C avec séparation par distillation d'un mélange d'hydroxy-3 tétrahydrofuranne et d'eau et addition simultanée d'une solution aqueuse de butane-triol-1,2,4, contenant au moins 60 % d'eau.